# NEUE EUROPÄISCHE PATENTSCHRIFT

(11) **EP 1 156 774 B2**
(45) Veröffentlichungstag und Bekanntmachung des Hinweises auf die Entscheidung über den Einspruch: **21.10.2009**
(45) Hinweis auf die Patenterteilung: 14.07.2004
(21) Anmeldenummer: 00903691.4
(22) Anmeldetag: 12.02.2000
(51) Int. Cl.: A61Q 5/00

(54) **ZUBEREITUNGEN ZUR BEHANDLUNG KERATINISCHER FASERN**
PREPARATIONS FOR TREATING KERATIN FIBRES
PREPARATIONS DESTINEES AU TRAITEMENT DE FIBRES KERATINIQUES

(30) Priorität: 23.02.1999 DE 19907715
(43) Veröffentlichungstag der Anmeldung: 28.11.2001
(73) Patentinhaber: Henkel AG & Co. KGaA, 40589 Düsseldorf (DE)
(72) Erfinder: SCHRÖDER, Thomas, D-22523 Hamburg (DE); BAUMSCHEIPER, Michael, D-25462 Rellingen (DE); POPPE, Elisabeth, D-22299 Hamburg (DE)
(74) Vertreter: Mathes, Nikolaus
(86) Internationale Anmeldenummer: PCT/EP2000/001158
(87) Internationale Veröffentlichungsnummer: WO 2000/049999

(56) Entgegenhaltungen:
- EP-A- 0 723 770
- WO- -95//05158
- WO- -97//25964
- WO-A-92/07546
- WO-A-95/01384
- WO-A-96/19966
- WO-A-97/25963
- DE-A- 4 327 699

## Beschreibung

Die Erfindung betrifft gelförmige Zubereitungen zur Behandlung, insbesondere zur Pflege, keratinischer Fasern, die als Schaum appliziert werden, die Verwendung dieser Zubereitungen zur Behandlung keratinischer Fasern sowie ein Verfahren zur Behandlung keratinischer Fasern.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten.

Dabei kommt der Pflege der Haare eine ständig wachsende Bedeutung zu. Dies betrifft nicht allein die seit jeher vornehmlich der Pflege der Haare dienenden Mittel wie Haarkuren und Haarspülungen. Auch für die Vermarktung von Produkten, die in erster Linie beispielsweise der Reinigung, Färbung oder Formgebung der Haare dienen, ist es zunehmend wichtig, pflegende Wirkungen ausloben zu können.

Schließlich ist es bei der zunehmenden Komplexität der Haarbehandlungsmittel wichtig, wenn die neu gefundenen Wirkstoffe oder Wirkstoffkombinationen sich durch hohe Kompatibilität mit möglichst vielen gängigen Bestandteilen von Haarbehandlungsmitteln auszeichnen oder sogar bisherige Kompatibilitätsprobleme umgehbar machen. In diesem Zusammenhang ist es wünschenswert, daß Komponente, die eine positive Wirkung auf dem Haar entfalten, zusätzlich die Aufgaben solcher Komponenten übernehmen, die bisher der Konfektionierung des Mittels dienen, sich aber auf dem Haar indifferent verhalten oder sogar einen negativen Einfluß auf die Eigenschaften des Haares ausüben.

Weiterhin ist es ein bleibendes Ziel, die Handhabung dieser Mittel durch den professionellen oder privaten Anwender möglichst einfach zu gestalten.

Unter Pflege von Haaren ist im weitesten Sinne sowohl die Reparatur, Minderung oder Prävention von Haarschädigungen, wie beispielsweise Brüchen, Oberflächendefekten und Spliß, als auch die Verringerung oder Vermeidung unerwünschter Eigenschaften des Haares, wie beispielsweise schlechte Naßkämmbarkeit, Verknotung der Haare, geringer Glanz und erhöhte elektrostatische Aufladung, zu verstehen.

Es wurde nun gefunden, daß sich durch eine spezielle Konfektionierungsform auf Basis bestimmter Rohstoffe Haarbehandlungsmittel formulieren lassen, die gegenüber den bekannten Mitteln hinsichtlich mehrerer Punkte deutlich verbessert sind.

Gegenstand der Erfindung sind daher gelförmige Zubereitungen zur Herstellung eines Schaumes für die Behandlung keratinischer Fasern, dadurch gekennzeichnet, daß die gelförmigen Zubereitungen auf wäßriger oder wäßrig-alkoholischer Basis formuliert sind und weiterhin enthalten
a) mindestens einen anionischen oder kationischen Gelbildner (G),
b) ein Treibmittel (T) und
c) mindestens einen Wirkstoff (W) aus der Gruppe, die von kationischen Tensiden, und Proteinhydrolysaten gebildet wird,
wobei der anionische Gelbildner ein Copolymer der im folgenden nach zu definierenden Art ist.

Unter keratinischen Fasern werden erfindungsgemäß Pelze, Wolle, Federn und insbesondere menschliche Haare verstanden.

Die erfindungsgemäßen Zubereitungen lassen sich als Schaum sehr gezielt auf das Haar aufbringen, so daß der "overspray", der z. B. zu unerwünschten Effekten auf der Kopfhaut oder Kleidung führen kann, äußerst gering ist. Die hervorragende Verteilung der Zubereitung auf dem Haar und die große Oberfläche aufgrund der Schaumstruktur ermöglichen ein hocheffektives Applizieren der Wirkstoffe. Aufgrund der durch den anionischen Gelbildner induzierten Gelstruktur des Mittels kann gewünschtenfalls auf reine Verdickungsmittel wie z. B. Celluloseether verzichtet werden. Diese Verdickungsmittel, die nur der Einstellung der vom Verbraucher bevorzugt akzeptieren Viskosität des Mittels dienen, bewirken in den meisten Fällen keinerlei positiven Effekt auf dem Haar selbst. Im Gegenteil können sie sogar beschwerend und belastend wirken und den Glanz des Haares in unerwünschter Weise herabsetzen. Weiterhin erfordert die Applikation in vielen Fällen kein vorheriges Schütteln der Spraydose.

Die erfindungsgemäßen Zubereitungen werden auf wäßriger oder wäßrig-alkoholischer Basis formuliert. Als alkoholische Komponenten kommen dabei niedere Alkanole sowie Polyole wie Propylenglykol und Glycerin zum Einsatz. Ethanol und Isopropanol sind bevorzugte Alkohole. Wasser und Alkohol können in der wäßrig alkoholischen Basis in einem Gewichtsverhältnis von 1 : 10 bis 10 : 1 vorliegen. Wasser sowie wäßrig-alkoholische Mischungen, die bis zu 50 Gew.-%, insbesondere bis zu 25 Gew.-%, Alkohol, bezogen auf das Gemisch Alkohol/Wasser, enthalten, können erfindungsgemäß bevorzugte Grundlagen sein.

Eine erste zwingende Komponente der erfindungsgemäßen Zubereitungen ist ein anionischer oder kationischer Gelbildner (G).

Erfindungsgemäß bevorzugt sind synthetische polymere Gelbildner.

Gemäß einer ersten, bevorzugten, Ausführungsform handelt es sich bei dem Gelbildner (G) um ein anionisches Copolymer aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vemetzt vorliegen, wobei als Vemetzungsagentien bevorzugt polyolefinisch ungesättigte Verbindungen wie Tetraallyloxythan, Allylsucrose, Allylpentaerythrit und Methylen-bisacrylamid zum Einsatz kommen. Ein solches Polymer ist in dem Handelsprodukt Sepigel® 305 der Firma SEPPIC-enthalten. Die Verwendung dieses Compounds, das neben der Polymerkomponente eine Kohlenwasserstoffmischung (C₁₃-C₁₄-Isoparaffin) und einen nichtionogenen Emulgator (Laureth-7) enthält, hat sich im Rahmen der erfindungsgemäßen Lehre als besonders vorteilhaft erwiesen.

Gemäß einer zweiten Ausführungsform handelt es sich bei dem Gelbildner (G) um einen kationischen synthetischen polymeren Gelbildner. Bevorzugte kationische Gruppen sind quartäre Ammoniumgruppen. Insbesondere solche Polymere, bei denen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden sind, haben sich als besonders geeignet erwiesen.

Homopolymere der allgemeinen Formel (I), 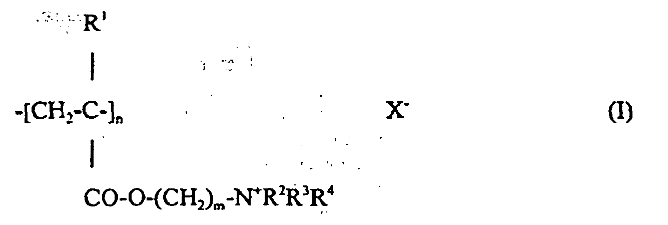 in der R¹ = -H oder -CH₃ ist, R², R³ und R⁴ unabhängig voneinander ausgewählt sind aus C₁₋₄-Alkyl-, -Alkenyl- oder -Hydroxyalkylgruppen, m = 1, 2, 3 oder 4, n eine natürliche Zahl und X⁻ ein physiologisch verträgliches organisches oder anorganisches Anion ist, sowie Copolymere, bestehend im wesentlichen aus den in Formel (I) aufgeführten Monomereinheiten sowie nichtionogenen Monomereinheiten, sind besonders bevorzugte kationische polymere Gelbildner. Im Rahmen dieser Polymeren sind diejenigen erfindungsgemäß bevorzugt, für die mindestens eine der folgenden Bedingungen gilt:
- R¹ steht für eine Methylgruppe
- R², R³ und R⁴ stehen für Methylgruppen
- m hat den Wert 2,

Als physiologisch verträgliches Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vemetzte, Poly(methacryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethem von Zuckern oder Zuckerderivaten wie Erythritol, Pentaerythritol, Arabitol, Monnitol, Sorbitol, Sucrose oder Glucose erfolgen. Methylenbisacrylamid ist ein bevorzugtes Vemetzungsagens.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare® SC 95 (ca. 50 % Polymeranteil, weitere Komponente: Mineralöl (INCI-Bezeichnung: Mineral Oil) und Tridecyl-polyoxypropylen-polyoxyethylen-ether (INCI-Bezeichnung: PPG-1-Trideceth-6)) und Salcare® SC 96 (ca. 50 % Polymeranteil, weitere Komponenten: Mischung von Diestem des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylenether (INCI-Bezeichnung: PPG- 1 -Trideceth-6) im Handel erhältlich.

Copolymere mit Monomereinheiten gemäß Formel (I) enthalten als nichtionogene Monomereinheiten bevorzugt Acrylamid, Methacrylamid, Acrylsäure-C₁₋₄-Alkylester und Methacrylsäure-C₁₋₄-Alkylester. Unter diesen nichtionogenen Monomeren ist das Acrylamid besonders bevorzugt. Auch diese Copolymere können, wie im Falle der Homopolymeren oben beschrieben, vemetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomeren in einem Gewichtsverhältnis von etwa 20:80 vorliegen, sind im Handel als ca. 50 %ige nichtwäßrige Polymerdispersion unter der Bezeichnung Salcare® SC 92 erhältlich.

Es ist erfindungsgemäß auch möglich, daß die Zubereitungen mehrere Gelbildner (G) enthalten. In diesem Fall ist es bevorzugt, Gelbildner gleicher Ionogenität einzusetzen.

Die erfindungsgemäßen Zubereitungen enthalten die Gelbildner (G) bevorzugt in Mengen von 0,1 - 5,0 Gew.-%, insbesondere in Mengen von 1,0 - 2,5 Gew.-%, bezogen auf die gesamte Zubereitung.

Gleichfalls ist es möglich, neben den zwingend notwendigen ionischen Gelbildnern (G) fakultativ weitere Gelbildner, insbesondere nichtionogene Gelbildner einzusetzen. Bevorzugte weitere Gelbildner sind in dieser Ausführungsform der Erfindung nichtionogene Polysaccharide wie Celluloseether sowie Xanthan-Gummi.

Die zweite zwingende Komponente der erfindungsgemäßen Zubereitungen ist das Treibmittel (T). Prinzipiell können als Treibmittel alle auf dem Gebiet der Kosmetik verwendeten, verflüssigbaren Gase eingesetzt werden. Dazu zählen beispielsweise niedere Alkane, Dimethylether sowie Fluorchlor- und Flurkohlenwasserstoffe. Bevorzugt werden das oder die Treibmittel so gewählt, daß die erfindungsgemäßen Zubereitungen auch über längere Zeiten bei den für Kosmetika üblichen Lagerbedingungen stabile homogene Gemsiche bilden. Bevorzugte Treibmittel sind niedere Alkane, insbesondere Propan, Butan, Isobutan sowie Mischungen dieser Alkane.

Die erfindungsgemäßen Zubereitungen enthalten die Treibmittel bevorzugt in Mengen von 1 - 20 Gew.-%, insbesondere in Mengen von 2 - 10 Gew.-%, bezogen auf die gesamte Zubereitung.

Die dritte zwingende Komponente der erfindungsgemäßen Zubereitungen ist ein pflegender Wirkstoff (W) aus der Gruppe, die von kationischen Tensiden, und Proteinhydrolysaten gebildet wird. Diese Wirkstoffe sind in den erfindungsgemäßen Zubereitungen bevorzugt in Mengen von 0,01 - 10 Gew.-%, insbesondere 0,2 - 5 Gew.-%, bezogen auf die gesamte Zubereitung, enthalten.

Erfindungsgemäß bevorzugt sind dabei kationische Tenside, insbesondere vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis(2-Palmitoyloxyethyl)dimethylammoniumchlorid, sowie Dehyquart® F-75 und Dehyquart® AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unterder Bezeichnung Tegoamid® S 18 im Handel erhältliche Stearamidopropyl-dimethylamin dar.

Proteinhydrolysate sind Produktgemische, die durch sauer, basisch oder enzymatisch katalysierten Abbau von Proteinen (Eiweißen) erhalten werden.

Erfindungsgemäß können Proteinhydrolysate sowohl pflanzlichen als auch tierischen Ursprungs eingesetzt werden.

Tierische Proteinhydrolysate sind beispielsweise Elastin-, Kollagen-, Keratin-, Seidenund Milcheiweiß-Proteinhydrolysate, die auch in Form von Salzen vorliegen können. Solche Produkte werden beispielsweise unter den Warenzeichen Dehylan® (Henkel), Promois® (Interorgana), Collapuron® (Henkel), Nutrilan® (Grünau), Gelita-Sol® (Deutsche Gelatine Fabriken Stoess & Co), Lexein® (Inolex) und Kerasol® (Croda) vertrieben.

Erfindungsgemäß bevorzugt ist die Verwendung von Proteinhydrolysaten pflanzlichen Ursprungs, z. B. Soja-, Mandel-, Erbsen-, Kartoffel- und Weizenproteinhydrolysate. Solche Produkte sind beispielsweise unter den Warenzeichen Gluadin® (Henkel), DiaMin® (Diamalt), Lexein® (Index) und Crotein® (Croda) erhältlich.

Wenngleich der Einsatz der Proteinhydrolysate als solche bevorzugt ist, können an deren Stelle gegebenenfalls auch anderweitig erhaltene Aminosäuregemische eingesetzt werden. Ebenfalls, wenngleich weniger bevorzugt, ist der Einsatz der Proteinhydrolysate in Form ihrer Fettsäure-Kondensationsprodukte sowie kationisch derivatisiert werden. Solche Handelsprodukte werden beispielsweise unter den Bezeichnungen Lamepon® (Henkel), Lexein® (Inolex), Crolastin® (Croda), Crotein® (Croda) und Croquat® vertrieben.

Die Proteinhydrolysate sind in den erfindungsgemäßen Zubereitungen bevorzugt in Mengen von etwa 0,01 - 3 Gew.-%, insbesondere 0,1 - 2 Gew.-%, bezogen auf die gesamte Zubereitung, enthalten.

Es hat sich weiterhin als vorteilhaft erwiesen, wenn die erfindungsgemäßen Zubereitungen zusätzlich zu den genannten zwingenden Komponenten ein filmbildendes nichtionogenes Polymer enthalten. Bevorzugte nichtionogene Filmbildner sind Polyvinylpyrrolidon und Vinylpyrrolidon/Vinylacetat-Copolymere, wie sie beispielsweise unter dem Warenzeichen Luviskol® im Handel erhältlich sind.

Hinsichtlich der Behandlungsart, für die die erfindungsgemäßen Zubereitungen als Schaum auf die Haare aufgebracht werden, bestehen keine prinzipiellen Einschränkungen.

Allerdings haben sich auf dem Haar verbleibende Zubereitungen als besonders wirksam erwiesen und können daher bevorzugte Ausführungsformen der erfindungsgemäßen Lehre darstellen. Unter auf dem Haar verbleibend werden erfindungsgemäß solche Zubereitungen verstanden, die nicht im Rahmen der Behandlung nach einem Zeitraum von wenigen Sekunden bis zu einer Stunde mit Hilfe von Wasser oder einer wäßrigen Lösung wieder aus dem Haar ausgespült werden. Vielmehr verbleiben die Zubereitungen bis zur nächsten Haarwäsche, d. h. in der Regel mehr als 12 Stunden, auf dem Haar.

Gemäß einer ersten Ausführungsform werden die erfindungsgemäßen Zubereitungen als Haarkur oder Haar-Conditioner formuliert. Die erfindungsgemäßen Zubereitungen gemäß dieser Ausführungsform können nach Ablauf dieser Einwirkzeit mit Wasser oder einem zumindest überwiegend wasserhaltigen Mittel ausgespült werden; bevorzugt werden-sie jedoch auch auf dem Haar belassen.

Gemäß weiteren Ausführungsformen kann es sich bei den erfindungsgemäßen Mitteln beispielsweise um reinigende Mittel wie Shampoos, pflegende Mittel wie Spülungen, festigende Mittel wie Haarfestiger, Schaumfestiger, Styling Gels und Fönwellen, dauerhafte Verformungsmittel wie Dauerwell- und Fixiermittel sowie insbesondere im Rahmen eines Dauerwellverfahrens eingesetzte Vorbehandlungsmittel oder Nachspülungen, farbverändernde Mittel wie Blondiermittel, Oxidationsfärbemittel, insbesondere im Rahmen eines oxidativen Färbeverfahrens verwendete Oxidationsmittelzubereitungen oder Nachspülungen, Tönungsmittel auf Basis direktziehender Farbstoffe und Tönungsfestiger handeln.

Der pH-Wert der erfindungsgemäßen Zubereitungen kann prinzipiell bei Werten von 2-11 liegen. Er liegt bevorzugt zwischen 2 und 7, wobei Werte von 3 bis 5 besonders bevorzugt sind. Zur Einstellung dieses pH-Wertes kann praktisch jede für kosmetische Zwecke verwendbare Säure oder Base verwendet werden. Üblicherweise werden als Säuren Genußsäuren verwendet. Unter Genußsäuren werden solche Säuren verstanden, die im Rahmen der üblichen Nahrungsaufnahme aufgenommen werden und positive Auswirkungen auf den menschlichen Organismus haben. Genußsäuren sind beispielsweise Essigsäure, Milchsäure, Weinsäure, Zitronensäure, Äpfelsäure, Ascorbinsäure und Gluconsäure. Im Rahmen der Erfindung ist die Verwendung von Zitronensäure und Milchsäure besonders bevorzugt. Bevorzugte Basen sind Ammoniak, Alkalihydroxide, Triethanolamin sowie N,N,N',N'-Tetrakis-(2-hydroxypropyl)-ethylendiamin.

Neben den erfindungsgemäß zwingend erforderlichen Bestandteilen können die erfindungsgemäßen Zubereitungen prinzipiell alle weiteren, dem Fachmann für solche kosmetischen Mittel bekannte Komponenten enthalten.

Weitere übliche Bestandteile der erfindungsgemäßen Zubereitungen können somit sein:
- nichtionogene Tenside wie beispielsweise Alkylphenolpolyglycolether, Fettsäurepolyglycolester, Fettsäureamidpolyglycolether, Fettaminpolyglycolether, alkoxylierte Triglyceride, wie insbesondere ethoxyliertes Rizinusöl, Alk(en)yloligoglucoside, Fettsäure-N-alkylglucamide, Polyolfettsäureester, Zuckerester, Sorbitanester und Polysorbate. Sofern die nichtionischen Tenside Polyglycoletherketten enthalten, können sie eine konventionelle oder eingeengte Homologenverteilung aufweisen.
- anionische Tenside, insbesondere Alkylsulfate, Alkylpolyglykolethersulfate und Ethercarbonsäuren mit 10 bis 18 C-Atomen in der Alkylgruppe und bis zu 12 Glykolethergruppen im Molekül, Seifen sowie Sulfobernsteinsäuremono- und - dialkylester mit 8 bis 18 C-Atomen in der Alkylgruppe und Sulfobernsteinsäuremono-alkylpolyoxyethyl-ester mit 8 bis 18 C-Atomen in der Alkylgruppe und 1 bis 6 Oxyethylgruppen,
- zwitterionische Tenside, insbesondere die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyldimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat,
- ampholytische Tenside wie beispielsweise N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkyl-aminoessigsäuren mit jeweils etwa 8 bis 18 C-Atomen in der Alkylgruppe,
- Anionische, zwitterionische und amphotere Polymere wie beispielsweise Vinylacetat/Crotonsäure-Copolymere, Vinylpyrrolidon/Vinylacrylat-Copolymere, Vinylacetat/Butylmaleat/Isobornyl-acrylat-Copolymere, Methylvinylether/Maleinsäureanhydrid-Copolymere und deren Ester, Acryl-amidopropyl-trimethylammoniumchlorid/Acrylat-Copolymere, Octylacrylamid/Methyl-methacry-lat/tert. Butylaminoethylmethacrylat/2-Hydroxypropylmethacrylat-Copolymere, Vinylpyrrolidon/Di-methylaminoethylmethacrylat/Vinylcaprolactam-Terpolymere sowie gegebenfalls derivatisierte Celluloseether,
- symmetrische und unsymmetrische, lineare und verzweigte Dialkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether und Di-n-dodecylether, n-Hexyl-n-octylether, n-Octyl-n-decylether, n-Decyl-nundecylether, n-Undecyl-n-dodecylether und n-Hexyl-n-Undecylether sowie Di-tertbutylether, Di-iso-pentylether, Di-3-ethyldecylether, tert.-Butyl-n-octylether, iso-Pentyl-n-octylether und 2-Methyl-pentyl-n-octylether,
- quaternierte Amine wie Methyl-1-alkylamidoethyl-2-alkylimidazolinium-methosulfat
- Entschäumer wie Silikone,
- Verdickungsmittel wie Agar-Agar, Guar-Gum, Alginate, Gelatine, Pektine sowie Polyacrylamide und deren Copolymere,
- Strukturanten wie Maleinsäure,
- Parfümöle, Dimethylisosorbid und Cyclodextrine,
- Lösungsvermittler, wie Ethanol, Isopropanol, Ethylenglykol, Propylenglykol, Gly cerin
   und Diethylenglykol,
- Farbstoffe zum Anfärben des Mittels,
- Antischuppenwirkstoffe wie Piroctone Olamine, Zink Omadine und Climbazol,
- weitere Substanzen zur Einstellung des pH-Wertes,
- Wirkstoffe wie Allantoin, Panthenol, Pyrrolidoncarbonsäuren, Vitamine und Bisabolol,
- Fettalkohole, insbesondere lineare und/oder gesättigte Fettalkohole mit 8 bis 22 C-Atomen, und Monoester der Fettsäuren mit Alkoholen mit 6 bis 24 C-Atomen,
- Pflanzenextrakte, insbesondere aus Eichenrinde, Brennessel, Hamamelis, Hopfen, Kamille, Klettenwurzel, Schachtelhalm, Lindenblüten, Mandel, Aloe Vera, Kokosnuß, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel,
- faserstrukturverbessernde Wirkstoffe, insbesondere Mono-, Di- und Oligosaccharide wie beispielsweise Glucose, Galactose, Fructose, Fruchtzucker und Lactose,
- konditionierende Wirkstoffe wie Paraffinöle, pflanzliche Öle, z. B. Sonnenblumenöl, Orangenöl, Mandelöl, Weizenkeimöl und Pfirsichkernöl sowie Phospholipide, beispielsweise Sojalecithin, Ei-Lecithin und Kephaline,
- Lichtschutzmittel, insbesondere derivatisierte Benzophenone, Zimtsäure-Derivate und Triazine,
- Konsistenzgeber wie Zuckerester, Polyolester oder Polyolalkylether,
- Fette und Wachse, wie Walrat, Bienenwachs, Montanwachs und Paraffine,
- Fettsäurealkanolamide,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere,
- Perlglanzmittel wie Ethylenglykolmono- und -distearat sowie PEG-3-distearat,
- Komplexbildner wie EDTA, NTA, β-Alanindiessigsäure und Phosphonsäuren,
- direktziehende Farbstoffe
- sogenannte Kuppler- und Entwicklerkomponenten als Oxidationsfarbstoffvorprodukte,
- Pigmente,
- Reduktionsmittel wie z. B. Thioglykolsäure und deren Derivate, Thiomilchsäure, Cysteamin, Thioäpfelsäure und α-Mercaptoethansulfonsäure,
- Oxidationsmittel wie Wasserstoffperoxid, Kaliumbromat und Natriumbromat sowie
- Antioxidantien.

Ein weiterer Gegenstand der Erfindung ist Verwendung der erfindungsgemäßen Zubereitungen nach einem der Ansprüche 1 bis 12 zur Behandlung keratinischer Fasern, insbesondere menschlicher Haare.

Ein dritter Gegenstand der Erfindung ist schließlich ein Verfahren zur Behandlung keratinischer Fasern, insbesondere menschlicher Haare, bei dem man eine Zubereitung gemäß einem der Ansprüche 1 bis 12 als Schaum auf die Faser aufbringt und bevorzugt auf dieser beläßt.

### Formulierungsbeispiele

Alle Mengenangaben sind, soweit nicht anders vermerkt, Gewichtsteile.

| **1. Schaumfestiger** | |
|---|---|
| Sepigel® 305¹ | 2,0 |
| Luviquat® FC 370² | 2,0 |
| Gluadin® Almond³ | 1,0 |
| Dow Corning® 939⁴ | 2,0 |
| Luviskol® VA 64 W⁵ | 1,0 |
| Ethanol | 25,0 |
| Propan/Butan-Gemisch⁶ | 3,0 |
| Wasser | ad 100 |

| | |
|---|---|
| ¹ Copolymer aus Acrylamid und 2-Acrylamido-2-methylpropansulfonsäure (INCI-Bezeichnung: Polyacrylamide (and) C₁₃-C₁₄Isoparaffin (and) Laureth-7) (SEPPIC) ² Vinylimidazoliummethochlorid-Vinylpyrrolidon-Copolymerisat (40 % Aktivsubstanz in Wasser; INCI-Bezeichnung: Polyquaternium-16) (BASF) ³ enzymatisch abgebautes Proteinhydrolysat aus süssem Mandelmehl (22 % Aktivsubstanz in Wasser; INCI-Bezeichnung: Aqua (and) Hydrolyzed Sweet Almond Protein (and) Sodium Benzoate (and) Phenoxyethanol (and) Methylparaben (and) Propylparaben) (HENKEL) ⁴ Emulsion eines aminofunktionellen Silikons (INCI-Bezeichnung: Amodimethicone (and) Trideceth-12 (and) Cetrimonium Chloride) (DOW CORNING) ⁵ Vinylpyrrolidon-Vinylacetat-Copolymer (60:40) (ca. 50 % Aktivsubstanz in Wasser; INCI-Bezeichnung: PVP/VA Copolymer) (BASF) ⁶ Massenverhältnis 1: 1 | |

| **2. Schaum-Conditioner** | |
|---|---|
| Salcare® SC96¹⁰ | 2,5 |
| Gafquat® 755N¹¹ | 1,5 |
| Gluadin® W20¹² | 1,0 |
| Dow Coming® 200 Fluid , 1cSt¹³ | 0,5 |
| Ethanol | 15,0 |
| Propan/Butan-Gemisch⁶ | 5,0 |
| Wasser | ad 100 |

| | |
|---|---|
| ¹⁰ N,N,N-Trimethyl-2[(methyl-1-oxo-2-propenyl)oxy]-Ethanaminiumchlorid-Homopolymer (50 % Aktivsubstanz; INCI-Bezeichnung: Polyquaternium-37 (and) Propylenglycol Dicaprilate Dicaprate (and) PPG-1 Trideceth-6) (ALLIED COLLOIDS) ¹¹ Dimethylaminoethylmethacrylat-Vinylpyrrolidon-Copolymer, mit Diethylsulfat quatemiert (19 % Aktivsubstanz in Wasser; INCI-Bezeichnung: Polyquaternium-11) (GAF) ¹² Weizenproteinhydrolysat (20 % Aktivsubstanz in Wasser; INCI-Bezeichnung: Aqua (and) Hydrolized Wheat Protein (and) Sodium Benzoate (and) Phenoxyethanol (and) Methylparaben (and) Propylparaben) (HENKEL) ¹³ Polydimethylsiloxan (INCI-Bezeichnung: Dimethicone) (DOW CORNING) ⁶ Massenverhältnis 1: 1 | |

| **3. Schaumfestiger** | |
|---|---|
| Sepigel® 305 | 1,5 |
| Luviquat® MS-370¹⁴ | 3,0 |
| Gluadin® Almond | 1,0 |
| Dow Corning® 939 | 2,0 |
| Luviskol® VA 55 I¹⁵ | 1,0 |
| Ethanol | 10,0 |
| Propan/Butan-Gemisch⁶ | 6,0 |
| Wasser | ad 100 |

| | |
|---|---|
| ¹⁴ Vinylpyrrolidon-Vinylimidazoliummethochlorid-Copolymer (ca. 40 % Aktivsubstanz; INCI-Bezeichnung: Polyquaternium-44) (BASF) ¹⁵ Vinylpyrrolidon-Vinylacetat-Copolymer (50:50) (ca. 50 % Aktivsubstanz in Isopropanol; INCI-Bezeichnung: PVP-VA Copolymer) (BASF) ⁶ Massenverhältnis 1: 1 | |

| **4. Schaum-Conditioner** | |
|---|---|
| Sepigel® 305 | 2,0 |
| Carbopol® 940¹⁶ | 0,25 |
| Polymer® JR 400¹⁷ | 1,0 |
| Gluadin® W 20 | 1,0 |
| Dow Corning® 939 | 1,0 |
| Dow Corning® 1401¹⁸ | 1,0 |
| Luviskol® VA 64 W | 1,0 |
| Ethanol | 15,0 |
| Propan/Butan-Gemisch⁶ | 6,0 |
| Neutrol® TE¹⁹ | ad pH 6,5 |
| Wasser | ad 100 |

| | |
|---|---|
| ¹⁶ Polyacrylsäure (INCI-Bezeichnung: Carbomer) (GOODRICH) ¹⁷ quaternierte Hydroxyethylcellulose (INCI-Bezeichnung: Polyquaternium-10) (AMERCHOL) ¹⁸ Dimethylcyclosiloxan-Dimethylpolysiloxanol-Gemisch (INCI-Bezeichnung: Cyclomethicone (and) Dimethiconol) (Dow Coming) ¹⁹ N,N,N`,N`-Tetrakis-(2-hydroxypropyl)-ethylendiamin (INCI-Bezeichnung: Tetrahydroxypropyl Ethylendiamine) (BASF) ⁶ Massenverhältnis 1: 1 | |

## Patentansprüche

1. Gelförmige Zubereitung zur Herstellung eines Schaumes für die Behandlung keratinischer Fasern, **dadurch gekennzeichnet, daß** die gelförmige Zubereitung auf wäßriger oder wäßrig-alkoholischer Basis formuliert ist und weiterhin enthält
a) mindestens einen anionischen oder kationischen Gelbildner (G), wobei der anionische Gelbildner (G) ein Copolymer ist, das besteht aus 70 bis 55 Mol% Acrylamid und 30 bis 45 Mol% 2-Acrylamido-2-methylpropansulfonsäure, wobei die Sulfonsäuregruppe ganz oder teilweise als Natrium-, Kalium-, Ammonium-, Mono-oder Triethanolammonium-Salz vorliegt,
b) ein Treibmittel (T) und
c) mindestens einen Wirkstoff (W) aus der Gruppe, die von kationischen Tensiden und Proteinhydrolysaten gebildet wird.

2. Zubereitung nach Anspruch 1, **dadurch gekennzeichnet, daß** es sich um einen synthetischen polymeren Gelbildner handelt.

3. Zubereitung nach Anspruch 2, **dadurch gekennzeichnet, daß** es sich bei dem Gelbildner (G) um einen kationischen Gelbildner mit quartären Ammoniumgruppen handelt.

4. Zubereitung nach Anspruch 3, **dadurch gekennzeichnet, daß** der kationische Gelbildner ausgewählt ist aus Homopolymeren der allegemeinen Formel (I), 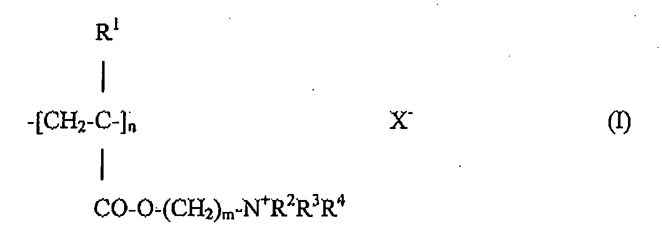 in der R¹ = -H oder -CH₃ ist, R², R³ und R⁴ unabhängig voneinander ausgewählt sind aus C₁₋₄-Alkyl-, -Alkenyl- oder -Hydroxyalkylgruppen, m = 1, 2, 3 oder 4, n eine natürliche Zahl und X⁻ ein physiologisch verträgliches organisches oder anorganisches Anion ist, und Copolymer, bestehend im wesentlichen aus den in Formel (I) aufgeführten Monomereinheiten sowie nichtionogenen Monomereinheiten.

5. Zubereitung nach einem der Ansprüche 1 bis 4, **dadurch gekennzeichnet, daß** das Treibmittel (T) ausgewählt ist aus der Gruppe, die von Propan, Butan und Isobutan sowie Mischungen dieser Alkane gebildet wird.

6. Zubereitung nach einem der Ansprüche 1 bis 5, **dadurch gekennzeichnet, daß** der Wirkstoff (W) ausgewählt ist aus kationischen Tensiden.

7. Zubereitung nach einem der Ansprüche 1 bis 6, **dadurch gekennzeichnet, daß** sie ein Silikon enthält,

8. Zubereitung nach einem der Ansprüche 1 bis 7, **dadurch gekennzeichnet, daß** sie einen nichtionogenen Filmbildner enthält.

9. Verwendung einer Zubereitung nach einem der Ansprüche 1 bis 8 zur Behandlung von keratinischen Fasern, insbesondere menschlichen Haaren.

10. Verfahren zur Behandlung von keratinischen fasern, insbesondere menschlichen Haaren, **dadurch gekennzeichnet, daß** eine Zubereitung nach einem der Ansprüche 1 bis 8 als Schaum auf das Haar aufgebracht wird.

11. Verfahren nach Anspruch 10, **dadurch gekennzeichnet, daß** die als Schaum aufgebrachte Zubereitung auf dem Haar verbleibt.

## Claims

1. A gel-form preparation for generating a foam for the treatment of keratinous fibres, **characterized in that** the gel-form preparation is water-based or water/alcohol-based and additionally contains
a) at least one anionic or cationic gel former (G), the anionic gel former (G) being a copolymer which consists of 70 to 55 mol-% acrylamide and 30 to 45 mol-% 2-acrylamido-2-methylpropanesulfonic acid, the sulfonic acid group being completely or partly present as a sodium, potassium, ammonium, mono- or triethanolammonium salt,
b) a blowing agent (T) and
c) at least one active component (W) from the group consisting of cationic surfactants and protein hydrolyzates.

2. A preparation as claimed in claim 1, **characterized in that** the gel (G) former is a synthetic polymeric gel former.

3. A preparation as claimed in claim 2, **characterized in that** the gel former (G) is a cationic gel former containing quaternary ammonium groups.

4. A preparation as claimed in claim 3, **characterized in that** the cationic gel former is selected from homopolymers corresponding to general formula (I): 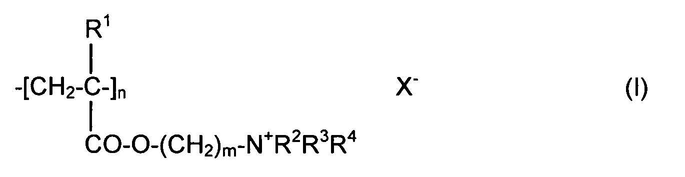 in which R¹ = -H or-CH₃, R², R³ and R⁴ independently of one another are selected from C₁₋₄ alkyl, C₁₋₄ alkenyl or C₁₋₄ hydroxyalkyl groups, m = 1, 2, 3 or 4, n is a natural number and X- is a physiologically compatible organic or inorganic anion,
and copolymers consisting essentially of the monomer units mentioned in formula (I) and nonionic monomer units.

5. A preparation as claimed in any of claims 1 to 4, **characterized in that** the blowing agent (T) is selected from the group consisting of propane, butane and isobutane and mixtures of these alkanes.

6. A preparation as claimed in any of claims 1 to 5, **characterized in that** the active component (W) is selected from cationic surfactants.

7. A preparation as claimed in any of claims 1 to 6, **characterized in that** it contains a silicone.

8. A preparation as claimed in any of claims 1 to 7, **characterized in that** it contains a nonionic film former.

9. The use of the preparation claimed in any of claims 1 to 8 for the treatment of keratinous fibres, more particularly human hair.

10. A process for treating keratinous fibres, more particularly human hair, **characterized in that** the preparation claimed in any of claims 1 to 8 is applied as a foam to the hair.

11. A process as claimed in claim 10, **characterized in that** the preparation applied as a foam remains on the hair.

## Revendications

1. Composition en forme de gel destinée à la préparation d'une mousse pour le traitement de fibres kératiniques, **caractérisée en ce que** la composition en forme de gel est formulée à partir d'une base aqueuse et/ou aqueuse-alcoolique et contient en outre
a) au moins un agent de formation de gel anionique ou cationique (G), l'agent de formation de gel anionique (G) étant un copolymère constitué par 70 à 55% en mole d'acrylamide et 30 à 45% en mole d'acide 2-acrylamido-2-méthylpropanesulfonique,le groupe acide sulfonique se trouvant totalement ou partiellement sous forme de sel de sodium, de potassium, d'ammonium, de monoéthanolammonium ou de triéthanolammonium,
b) un agent gonflant (T) et
c) au moins une substance active (W) du groupe formé par les agents tensioactifs cationiques, et les hydrolysats de protéines.

2. Composition selon la revendication 1, **caractérisée en ce qu'**il s'agit d'un agent de formation de gel polymère synthétique.

3. Composition selon la revendication 2, **caractérisée en ce qu'**il s'agit, pour l'agent de formation de gel (G) d'un agent de formation de gel cationique présentant des groupes ammonium quaternaire.

4. Composition selon la revendication 3, **caractérisée en ce que** l'agent de formation de gel cationique est choisi parmi les homopolymères de formule générale (I) 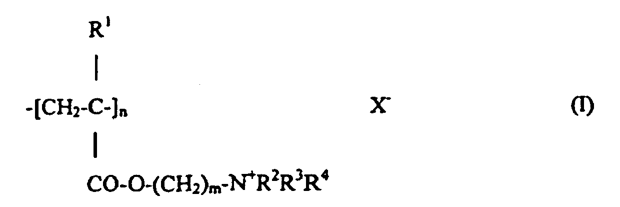 dans laquelle R¹ = -H ou -CH₃, R², R³ et R⁴ sont choisis, indépendamment l'un de l'autre, parmi les groupes alkyle en C₁ à C₄, alcényle en C₁ à C₄ ou hydroxyalkyle en C₁ à C₄, m = 1, 2, 3 ou 4, n est un nombre naturel et X-représente un anion physiologiquement acceptable, organique ou inorganique et les copolymères essentiellement constitués des unités monomères indiquées dans la formule (I) et d'unités monomères non ionogènes.

5. Composition selon l'une quelconque des revendications 1 à 4, **caractérisée en ce que** l'agent gonflant (T) est choisi dans le groupe formé par le propane, le butane et l'isobutane ainsi que les mélanges de ces alcanes.

6. Composition selon l'une quelconque des revendications 1 à 5, **caractérisée en ce que** la substance active (W) est choisie parmi les agents tensioactifs cationiques.

7. Composition selon l'une quelconque des revendications 1 à 6, **caractérisée en ce qu'**elle contient un silicone.

8. Composition selon l'une quelconque des revendications 1 à 7, **caractérisée en ce qu'**elle contient un agent de formation de film non ionogène.

9. Utilisation d'une composition selon l'une quelconque des revendications 1 à 8, pour le traitement de fibres kératiniques, en particulier les cheveux humains.

10. Procédé pour le traitement de fibres kératiniques, en particulier les cheveux humains, **caractérisé en ce qu'**une composition selon l'une quelconque des revendications 1 à 8 est appliquée sous forme de mousse sur les cheveux.

11. Procédé selon la revendication 10, **caractérisé en ce que** la composition appliquée sous forme de mousse reste sur les cheveux.
